# EUROPEAN PATENT APPLICATION

(11) **EP 4 775 594 A1**
(43) Date of publication of application: **15.07.2026**
(21) Application number: 24799477.5
(22) Date of filing: 23.08.2024
(51) Int. Cl.: C07K 16/28, C07K 16/30

(54) **BISPECIFIC ANTIBODIES THAT BIND CD3 AND THE GANGLIOSIDE NGCGM3**

(30) Priority: 08.09.2023 CU 20230043
(71) Applicant: Centro de Inmunologia Molecular, Playa, La Habana 11300 (CU)
(72) Inventor: HERNÁNDEZ GARCÍA, Tays, La Habana 11300 (CU); PLASENCIA IGLESIAS, Claudia Aracelis, La Habana 11300 (CU); DIEGO MATOS, Ana Karla, Lisa La Habana 17100 (CU); LEÓN MONZÓN, Kalet, La Lisa La Habana 17100 (CU); ZEKRI, Latifa, 72074 Tübingen (DE); JUNG, Gundram, 72108 Rottenburg (DE)
(74) Representative: Capitán García, Maria Nuria
(86) International application number: PCT/CU2024/050006
(87) International publication number: WO 2025/051309

(57) **Abstract**

**SUMMARY**

The present invention is related to the field of Biotechnology and Immuno-oncology. Bispecific antibodies comprising an antibody, antibody fragment or single chain variable fragment that recognize the NGcGM3 ganglioside on tumor cells and an antibody, antibody fragment or single chain variable fragment that recognize the CD3 antigen on human immune effector cells are described. These bispecific antibodies are characterized by their ability to mediate selective cytotoxicity on NGcGM3-positive tumor cells, and not on normal cells that can express the ganglioside, and allow the recruitment of not only T lymphocytes, but also NKT. The bispecific antibodies of the present invention and the nucleic acids that encode them are useful in the treatment of lymphoproliferative disorders and solid tumors that express NGcGM3.

## Description

### FIELD OF TECHNIQUE

The present invention is related to the field of Biotechnology and Immuno-oncology. Particularly, it describes bispecific antibodies that recognize the NGcGM3 antigen, expressed in tumors, and the CD3 molecule, and have the capacity to recruit effector functions of T, NKT and Tγδ cells.

### BACKGROUND

T-cell-engaging bispecific antibodies (BAbs-T) have progressively been consolidated as one of the most promising alternatives for the re-direction of effector cells of the immune system to the tumor, where they recognize an antigen on the surface of malignant cells (Wu, Z. and N. Cheung (2018) Pharmacology therapeutics 182:161-175). Thus, the recruitment of T lymphocytes towards tumor cells, independently of the specific recognition of antigen by the T cell receptor, is a very attractive and novel approach. One of the challenges of this type of therapy is the appropriate selection of the tumor antigen against it is directed to. Tumor-specific antigens, resulting from genetic and epigenetic changes, are the ones that selectively target tumors the most (Apavaloaei, A., et al. (2020) Cancers 12 (9):2607). Unfortunately, many of them are intracellular and not accessible to standard BAbs-T. Tumor-selective antigens include those overexpressed by the tumor or located differentially with respect to normal tissues, and are also exploited in the therapy based on BAbs-T, but do not exclude the appearance of potential adverse effects.

Specifically, BAbs-T against solid tumors often show safety problems associated with cytokine release syndrome (CRS) and damage to normal tissues that express the antigen. Preliminary results with BAbs-T in solid tumors have reported generally higher CRS rates (19-91%) than those intended for the treatment of hematological tumors such as blinatumumab (7%-15%) (Bendell, J. C., et al. (2020); J Clin Oncol 38: 55-52; Borghaei, H., et al. (2020); Middleton, M. R., et al. (2020); Clinical Cancer Research 26 (22): 5869-5878; Tran, B., et al (2020). Annals of Oncology 31: S507). On target off tumor toxicity has been a limiting factor in the use of BAbs-T against these types of tumors (Kobold, S., et al. (2018) Frontiers in Oncology 8:285).

The NGcGM3 ganglioside constitutes a widely studied target, given its expression in human tumors of different locations and little presence in normal tissues (Bardor, M., et al. (2005) Journal of Biological Chemistry 280(6): 4228-4237), based on which several therapies have been conceived (Blanco, R., et al. (2011) International Scholarly Research Notices; Lahera, T., et al. (2014) Oncol). These include the development of vaccines, such as Glycovax (Carr, A., et al. (2003). Journal of Clinical Oncology 21(6): 1015-1021), monoclonal antibodies (MAbs) such as 14F7, which is characterized by a fine recognition of the GM3 ganglioside, capable of distinguishing its N-glycolylated variant (which it recognizes) from the N-acetylated version (which it does not recognize) (Carr, A., et al. (2002). Hybridoma hybridomics 21(6): 463 -468; Casadesús, A. V., et al. (2013) Glycoconjugate 30: 687-699), and fragments derived from it such as 3FM and 8Bhl (Rojas, G., et al. (2004). Journal of immunological methods 293(1-2): 71-83). The 14F7 antibody is capable of inducing an oncosis-like cell death in mouse L1210 tumor cells (lymphoid origin), but not in normal mouse lymphocytes (B and TCD4+). These differences in cytotoxicity could be attributed to the differential distribution of the NGcGM3 ganglioside in the lipid rafts of the plasma membrane, between normal and tumor cells (Roque-Navarro, L., et al. (2008) Molecular Cancer Therapeutics 7(7): 2033-2041). However, these differences between tumor and normal cells in terms of ganglioside distribution do not have to determine a similar selectivity for other types of death induced by the 14F7 binding domain and involving the participation of effector cells.

Given the non-protein nature of NGcGM3, therapeutic approaches such as MAbs and vaccines are inefficient in recruiting the T cell response and its cytotoxic capacity to tumors that express this ganglioside. This raises the need to develop therapies that mediate T lymphocyte cytotoxicity on NGcGM3-positive tumor cells. To date, no BAb-T specific for this ganglioside has been described.

Since the generation of the first BAb-T, dissimilar formats have been described, ranging from the smallest designs to those based on complete IgG structures (Godar, M. et al. (2018). Expert Opinion on Therapeutic patents 28(3):251-276).

Although the format of choice can influence the functionality of these antibodies, the central role of the target molecule in the potency and safety of BAbs-T is well recognized (Li, H., et al. (2020) Cellular molecular immunology 17(5): 451-461). This is influenced by the location of the recognized epitope and the dimensions of the target molecule. These features play a fundamental role in the efficiency of the formation of the immunological synapse and subsequent activation of T lymphocytes, being determinant in the cytotoxic capacity of this type of antibodies (Li, J., et al. (2017). Cancer cell 31(3): 383-395). In summary, the nature of the target is critical to guarantee the effectiveness of a BAb-T, as not just any epitope is capable of successfully recruiting the action of a T lymphocyte through a BAb-T directed against it.

Although CD3-specific BAb-T can theoretically recruit the cytotoxic activity not only of T lymphocytes, but also of NKT and Tγδ, practically no examples demonstrating this capacity are documented. A BAb-T specific for the human PDL1 molecule demonstrated its ability to mediate cytotoxicity on PDL1+ tumor cells by peripheral blood NKT cells from a healthy human donor (Horn, L. A., et al. (2017) Oncotarget 8(35): 57964). This property has not been described to date for any BAb-T against any other molecular target.

Considering the set of backgrounds described above, the inventors of the present application generated BAbs-T for cancer therapy, which recognize the NGcGM3 ganglioside, the first BAbs-T described with this antigenic specificity.

The anti-NGcGM3 BAbs-T of the present embodiment surprisingly achieve potent activity in vitro, with the use of human lymphocytes, without the need to activate them previously, which especially demonstrates their potent cytotoxic effect.

Additionally, these BAbs-T are characterized by their ability to mediate selective cytotoxicity on NGcGM3-positive tumor cells, and not on normal cells, despite the fact that they can express this ganglioside, which provides a margin of safety and minimizes the on target-off tumor effect. As a result, molecules with antitumor properties with great specificity for malignant cells are obtained.

Furthermore, these BAbs-T against NGcGM3 allow the recruitment of not only T lymphocytes, but also NKT, and the induction of tumor cell lysis, and are the first BAbs-T with such specificity showing this property.

### BRIEF DESCRIPTION OF THE INVENTION

In one embodiment the present invention relates to BAbs-T comprising an antibody, antibody fragment or single chain variable fragment (scFv) that recognizes the NGcGM3 ganglioside on tumor cells and an antibody, antibody fragment or scFv that recognizes the human CD3 molecule. Particularly, the antibody that recognizes NGcGM3 is a human IgG whose sequence is selected from the group comprising SEQ ID NO. 1-4 or variants of these with more than 90% identity with respect to these sequences.

These BAbs-T may have a format that is selected from the group comprising: TandAb, DART, DART-Fc, DuoBody, CrossMab, KiH, BiTE, Triomab, IgG-scFv.

In a particular embodiment the heavy chain of the antibody that recognizes NGcGM3 is fused directly or through a linker to antibodies, antibody fragments or scFv specific for human CD3.

In another embodiment the antibodies, antibody fragments or scFv that recognize the human CD3 molecule have the sequences that are selected from the group comprising SEQ ID NO. 5-7, or variants of these with more than 90% identity with respect to said sequences.

In another particular embodiment the antibodies, antibody fragments or scFv that recognize NGcGM3 have a heavy chain variable region that is selected from the group comprising SEQ ID NO. 10 and SEQ ID NO. 15, or variants thereof with more than 90% identity with respect to these sequences and the antibodies, antibody fragments or scFv that recognize NGcGM3 have a light chain variable region that is selected from the group comprising SEQ ID NO. 11-14 or variants of these with more than 90% identity with respect to these sequences.

In another embodiment, the present invention is related to pharmaceutical compositions that comprise as active ingredients the BAbs-T described here and a pharmaceutically acceptable carrier.

In a further embodiment, the present invention relates to the use of BAbs-T in the treatment of lymphoproliferative disorders and solid tumors that express NGcGM3. Particularly, with the use of the nucleic acid encoding the BAbs-T in the gene therapy of tumors that express NGcGM3, said gene therapy is based on the injection of mRNA or transducer particles encoding the BAbs-T.

In another embodiment, the present invention relates to a method of treating a subject in need that comprises the administration by subcutaneous, intravenous, intradermal, intramuscular, intratumoral or intraperitoneal route of the BAbs-T that are disclosed herein in a range of doses between 20ug-10mg.

### DETAILED DESCRIPTION OF THE INVENTION

### Description of BAbs-T

The present invention relates to bispecific antibodies or multifunctional fusion proteins comprising an antibody, antibody fragment or scFv that recognizes the NGcGM3 ganglioside on tumor cells, and an antibody, antibody fragment or scFv that recognizes the human CD3 molecule.

The term T-cell-engaging bispecific antibody (BAb-T) used in the present invention refers to a category of bispecific antibodies that are capable of engaging and recruiting the cytotoxic action of T and NKT cells, to a tumor cell that expresses a specific antigen. The terms fusion protein and BAb-T are used interchangeably in the present invention. These BAbs-T can have different formats that are well known to a person skilled in the art, who can use the variable domains and/or the sets of CDRs described here and obtain the different formats by routine techniques (Godar, M. et al. (2018). Expert Opinion on Therapeutic patents 28(3):251-276). The formats of the fusion proteins of the present invention guarantee the recruitment, activation and cytotoxic capacity of T lymphocytes and NKT cells towards tumor cells.

According to one of the embodiments of the invention, the BAbs-T described here adopt a complete IgG-based structure.

In a particular embodiment, the IgG backbone is based on a human IgG1 that contains a set of modifications that abolish or reduce Fc^{γ}R binding and complement fixation, and decrease its potential to recruit effector functions associated with its Fc region. This modified Fc prevents the cross-linking of the Fcy-CD3 receptors, caused by the binding of the antibodies to a site different to the tumor target and, consequently, prevents the unwanted action on other cells of the immune system mediated by T cells, which has limited the efficacy of BAbs-T (Labrijn, A. F., et al. (2019) Nature reviews Drug discovery 18(8): 585-608).

Particularly, the human IgG1 of these BAbs-T has modifications in the CH2 domain (SEQ ID NO. 1) that abrogate or decrease Fc^{γ}R binding and complement fixation (Wines, B. D., et al. (2000) The Journal of Immunology 164(10): 5313-5318; Armour, K. L., et al. (2003) Molecular immunology 40(9):585-593; Sazinsky, S. L., et al. (2008) Proceedings of the National Academy of Sciences 105( 51): 20167-20172; Schlothauer, Herter et al. (2016). Protein Engineering, Design 29(10): 457-466).

Optionally, the modified human IgG1 backbone has the N297X mutation (SEQ ID NO. 2), where X is selected from the group of amino acids comprising: alanine, glycine and glutamine). This mutation eliminates the classic glycosylation site of the Fc region of human IgG1 (Chao, D. T., et al. (2009) Immunological investigations 38(1): 76-92), which results in obtaining an aglycosylated immunoglobulin in said region, which does not have the ability to bind to Fcγ receptors (Wang, L.-X., et al. (2019). Annual review of biochemistry 88: 433-459). Additionally, the IgG backbone contains those variants with more than 90% identity with SEQ ID NO. 1 and 2.

In a particular embodiment the human IgG backbone is an IgG2 (SEQ ID NO. 3, Uniprot P01859) or an IgG4 (SEQ ID NO. 4, Uniprot P01861) which do not have the ability to bind to Fcγ receptors (Vafa, O., et al. (2014) Methods 65(1): 114-126; Gillies, S. D., et al. (1999) Cancer research 59(9):2159-2166; Newman, R., et al. (2001) Clinical Immunology 98(2):164-174), as well as variants that have more than 90% identity with said sequences.

Optionally, the design of the BAbs-T object of the present invention is that of a tetravalent molecule based on complete IgG with minimized or no effector functions, with two binding domains to the NGcGM3 ganglioside, and two binding domains to human CD3. In another embodiment, the BAbs-T against NGcGM3 obtained in the present invention are characterized by having an anti-CD3 scFv linked directly or through a linker of 10-27 amino acid residues, to the carboxyl end of the heavy chains of the anti-NGcGM3 antibody (IgG-scFv format). Particularly, the scFvs of anti-CD3 antibodies humanized UCHT1 (Zekri, L., et al. (2021) EMBO molecular medicine 13(2):e11902, SEQ ID NO. 5), humanized OKT3 (Adair, J. R. , et al (1994) Human Antibodies 5(1-2):41-47; SEQ ID NO. 6) or L2K (US9611325B2, SEQ NO. ID 7), or variants of these with more than 90% identity with respect to these sequences are bound.

Additionally, the BAbs-T of the present invention can adopt formats already described in the prior art such as BiTE (WO2005061547, Frankel and Baeuerle, 2013), Triomab (WO1995033844, Lindhofer, H., et al. (1995) Journal of immunology 155(1):219-225; Tandem diAbody (TandAb) (Reusch, U., et al. (2015). MAbs 7(3): 584-604; Dual Affinity Retargeting (DART)/Dual affinity Retargeting-Fc (DART-Fc) (Chichili G. R. et al. (2015) Sci Transl Med 7(289): 289ra82), DuoBody (Gaudet, F., et al. (2016) Blood 128(22):2824) and CrossMab and/or Knobs into holes (KiH) (WO201326833; Bacac, M., et al. (2016) Clinical Cancer Research 22(13):3286-3297).

The BAbs-T described in the present invention recognize the NGcGM3 ganglioside with an affinity in the range (KD between 10⁻⁷ and 10^{-10M}), and bind to the human CD3 molecule with affinities with KD in the range 10⁻⁷ and 10^{-10M}), which allow the activation of T cells or others that express the CD3 cluster.

The BAbs-T described here recruit T cells through their CD3-specific scFv domain. Furthermore, these antibodies exhibit the property of recruiting and activating NKT cells that mediate the lysis of tumor cells expressing NGcGM3.

In a particular embodiment, the constant region of the light chain of the BAbs-T is characterized by belonging to the kappa (Uniprot P01834) or human lambda isotype (Uniprot P0CG04) and has the sequence that corresponds to SEQ ID 8 and SEQ 9, respectively, and variants that have more than 90% identity with said sequences.

On the other hand, the variable regions of the heavy chain of the antibody can be murine, humanized or fully human and come from immunoglobulins with the capacity to recognize the NGcGM3 ganglioside. Said variable regions are identified, but are not limited, to SEQ ID NO 10, which comprises variants containing any of the following mutations:
Position 5: Q por V
Position 9: N por A
Position 11: L por V
Position 12: A por V
Position 18: M por V
Position 19: K por R
Position 120: M por V
Position 40: R por A
Position 42: D por G
Position 48: I por V

Furthermore, the variable regions of the antibody light chain can be murine, humanized or fully human. Such variable regions are identified, but are not limited, to SEQ ID NO 11 which includes variants containing any of the following mutations:
Light chain
Position 39: R por K
Position 40: T por P
Position 41: H por G
Position 42: E por Q
Position 58: I por V

Additionally, these light chain variable regions include SEQ ID NO 12 and SEQ ID NO 13.

### Pharmaceutical compositions

The BAbs-T object of the present invention can be found as an active ingredient, forming part of different pharmaceutical compositions appropriate for them, and a pharmaceutically acceptable vehicle. The concentrations of the active ingredient in said pharmaceutical compositions are in the range of 0.5 mg/ml to 20 mg/ml, preferably 1 mg/ml to 10 mg/ml, or lyophilized.

Pharmaceutically acceptable carriers include, but are not limited to: saline, pH neutral phosphate buffered saline, and others similar. Other buffering agents, dispersing agents, and non-toxic inert substances suitable for delivery to a patient may be included in the compositions of the present invention. The compositions may be solutions suitable for administration, and are normally sterile and free of undesirable particles.

### Therapeutic application and treatment methods

The present invention relates to the use of the BAbs-T disclosed herein in the treatment of NGcGM3 positive tumors. In particular, in the treatment of lymphoproliferative disorders such as chronic lymphocytic leukemia (CLL), and multiple myeloma, as well as advanced-stage solid tumors that express NGcGM3, such as head and neck tumors, brain tumors, adult and pediatric gliomas, pancreatic cancer, tumors of the esophagus, breast, non-small cell lung cancer and nasopharyngeal, stomach, bladder, colorectal, and melanoma tumors. Additionally, it is related to the use of gene therapy approaches based on the injection of mRNA or transducer particles encoding the BAbs-T of the present invention, by subcutaneous, intramuscular or intratumoral routes, for the treatment of the above-mentioned malignancies.

In a further embodiment, the present invention relates to a method of treating a subject in need that comprises the administration by subcutaneous, intravenous, intradermal, intramuscular, intratumoral or intraperitoneal route of pharmaceutical compositions that contain as active ingredient any of the BAbs-T that are described here in a dose range between 20ug-10mg. Particularly, the administration of said pharmaceutical compositions is carried out from one to 13 cycles, between 1 and 7 infusions per cycle, in cycles of 21 or 28 days.

The possible combination with other immunotherapies in patients with various types of cancer is supported by the immunomodulatory properties of these molecules, in addition to the cytotoxic action they mediate on tumor cells. Likewise, it is possible their combination with other immunomodulators and with classic oncotherapies such as chemotherapy and radiotherapy. In this way, the mentioned BAbs-T aim to constitute a therapeutic front in the treatment of cancer. This effect would be associated with the selective recruitment of the cytotoxic action of immune effector cells such as T and NKT lymphocytes on tumor cells that express NGcGM3, which leads to a delay in tumor growth and greater survival of the treated individuals. Added to this is the ability to induce a controlled environment of inflammatory cytokines that can contribute to the activation of other immune effectors and thus enhance the antitumor effect. At the same time, it is important to highlight the low levels of toxicity that they show in animal models, on healthy tissues that express the NGcGM3 ganglioside, which supports a good safety profile in their use in patients.

All this translates into greater hope and quality of life in treated patients.

### BRIEF DESCRIPTION OF THE FIGURES

**Figure 1****.** Representation of the format of the anti-NGcGM3 BAbs-T.
**Figure 2****.** Sodium dodecyl sulfate polyacrylamide gel electrophoresis (SDS-PAGE) of the antibodies 14F7hT-UCHT1 (A, lanes 1 and 2), 3Fm-UCHT1 (A, lanes 3 and 4), 14F7hT (A, lanes 5 and 6; B and C, lane 1) and 14F7hT-2c11 (B and C, lane 2). Non-reducing conditions (A: lanes 1, 3, 5; B: lanes 1 and 2). Reducing conditions (A: lanes 2, 4, 6; C: lanes 1 and 2).
**Figure 3****.** Recognition of NGcGM3 on the surface of the P3X63 and L1210 tumor cell lines, by the BAbs-T 14F7hT-UCHT1 and 3Fm-UCHT1 (A), and 14F7hT-2c11 (B), and recognition of CD3 on the surface of the Jurkat cell line by the BAbs-T 14F7hT-UCHT1 and 3Fm-UCHT1 (C).
**Figure 4****.** Cytotoxic activity on P3X63 tumor cell line, mediated by the BAbs-T 14F7hT-UCHT1 (A and B), 3Fm-UCHT1 (A) and 14F7hT-2c11 (C), in the presence of effector cells.
**Figure 5****.** Cytotoxic activity of mouse NKT cells on the P3X63 tumor cell line, mediated by the 14F7hT-2c11 antibody. Purified NKT cells (A) and the NKT hybridoma (B) were used as effectors.
**Figure 6****.** Antitumor effect of 14F7hT-2c11 in a BALB/c-X63 therapeutic model, measured on day 7 after tumor growth.
**Figure 7****.** Activation of T cells by 14F7hT-UCHT1 antibodies, in the presence of the P3X63 tumor cell line, CD8+ (A) and CD4+ (B).
**Figure 8****.** Induction of T lymphocyte proliferation mediated by the BAbs-T 14F7hT-UCHT1 (A and B) and 14F7hT-2c11 (C), in the presence of the P3X63 tumor cell line.
**Figure 9****.** Specificity of the cytotoxic effect on tumor cells mediated by human effector cells and 14F7hT-UCHT1.
**Figure 10****.** Absence of cytotoxicity of BAbs-T 14F7hT-2c11 (A) and 14F7hT-UCHT1 (B) on normal cells (mouse lymphocytes).
**Figure 11****.** Evaluation of the in vivo activation of T cells mediated by the BAbs-T 14F7hT-UCHT1 (A) and 14F7hT-2c11 (B), in the presence of normal tissues that express this ganglioside, according to evaluation of IFNγ in the serum of treated animals.
**Figure 12****:** Evaluation of the in vivo toxicity of BAbs-T 14F7hT-UCHT1 according to measurement of body weight (A) and relative organ weight (B).
**Figure 13****:** Evaluation of the in vivo toxicity of BAb-T 14F7hT-2c11 according to body weight measurement.

The present invention is further elaborated with the following examples and drawings. However, these examples should not be interpreted as limiting the scope of the invention.

### EXAMPLES

### Example 1. Design and obtaining of the genetic constructs encoding BAbs-T.

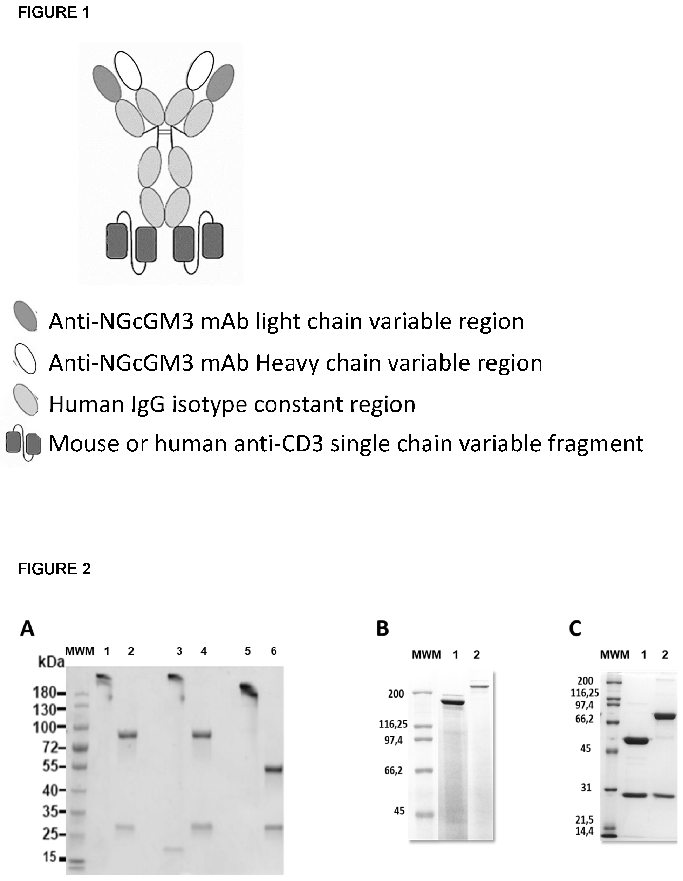

Human BAbs-T consisting of multifunctional molecules were designed, with a format based on complete IgG (IgG-scFv), with bivalent binding to NGcGM3 and human CD3. Specifically, the BAbs-T 14F7-UCHT1, 3FM-UCHT1 and 8Bhl-UCHT1 correspond to a format as represented in Figure 1.

To model the activity of human BAbs-T in immunocompetent mice, BAb-T 14F7-2c11 was designed, with identical specificity for the tumor antigen and the same format as human BAbs-T. With bivalent binding to NGcGM3 and mouse CD3, its format is depicted in Figure 1.

The antigen binding domains of the BAbs-T 14F7hT-UCHT1 and 14F7hT-2c11 contain the variable regions (VL and VH) of the 14F7hT MAb (Fernández-Marrero, Y., et al. (2011) Immunobiology 216(12) :1239-1247, SEQ ID NOs.14 and 15, respectively); that of 3Fm-UCHT1 conserves the VH region of the 14F7hT (SEQ. ID NO 15) and shares the VL region of the scFv3Fm fragment (SEQ. ID NO. 12), while that of 8Bhl-UCHT1 conserves the VH region of the 14F7hT MAb (SEQ. ID NO. 15) and shares the VL region of the scFv8Bhl fragment (SEQ. ID NO. 13). The BAbs-T 14F7hT-UCHT1, 3Fm-UCHT1, 8Bhl-UCHT1 have fused to the C-terminal of the heavy chain, the scFv fragment of the humanized UCHT1 MAb, specific for human CD3 (Zekri, L., et al. (2021) EMBO molecular medicine 13(2):e11902, SEQ. ID 5), while 14F7hT-2c11 has the scFv 2c11, specific for the mouse CD3 molecule, fused to the same site (SEQ. ID 16, Fernandes, R. A., et al. (2012). Journal of Biological Chemistry 287 (16): 13324-13335). Additionally, the human IgG1 backbone used in the four BAbs-T is identified with SEQ. ID NO. 1. On the other hand, the light chain of the anti-NGcGM3 BAbs-T 14F7-UCHT1, 3FM-UCHT1, 8Bhl-UCHT1 and 14F7hT-2c11 has the human C kappa constant region (SEQ. ID NO. 8).

To obtain the genetic constructs that encode the BAbs-T light chains, the VL3Fm and VL14F7hT genes were cloned into the pGH1.2_VκPSMA_Cκh vector. The genetic constructs pGH1.2_VL3Fm_Cκh and pGH1.2_VL14F7hT_Cκh were thus obtained. The first was used to obtain 3FM-UCHT1, while the second was used to generate the BAbs-T 14F7-UCHT1 and 14F7hT-2c11. To obtain the genetic construct that encodes the heavy chain of BAbs-T14F7-UCHT1 and 3FM-UCHT1, the VH gene of AcM 14F7hT was cloned in the vector pGH1.2-IgGscFv_PSMA_UCHT1 (modified IgG1h-scFvUCHT1). To obtain the genetic construct that encodes the heavy chain of 14F7hT-2c11, the scFv 2c11 gene from the construct pGH1.2-IgGscFv_10B3_2c11 was cloned into the vector pGH1.2-IgGscFv_14F7hT_UCHT1, previously obtained.

### Example 2. The anti-NGcGM3 BAbs-T are expressed as intact and functional proteins.

The transient expression of BAbs-T was carried out in ExpiCHO-S cells in suspension, and using the genetic constructs described above. Ten days after transfection, the recombinant molecules contained in the supernatants were purified by a sequential protocol, consisting of a first step of protein A affinity chromatography, followed by molecular exclusion chromatography.

Next, the size and integrity of the BAbs-T were confirmed by SDS-PAGE and subsequent staining with Coomassie Blue. For BAbs-T 14F7hT-UCHT1 and 3Fm-UCHT1, SDS-PAGE was performed on 10% polyacrylamide gels, using reducing and non-reducing conditions (Figure 2A). For the 14F7hT-2c11 antibody, SDS-PAGE was performed on 7.5% (Figure 2B) and 12% (Figure 2C) polyacrylamide gels, under non-reducing and reducing conditions, respectively. The 14F7hT MAb was used as a monospecific antibody control. As seen in Figure 2, the non-reducing conditions showed that the purified BAbs-T yielded a band that migrated to the expected size, equivalent to approximately 204 kDa. The 14F7-hT MAb, as expected, had greater electrophoretic mobility according to its molecular weight close to 150 kDa. The reducing conditions of the experiment showed the presence of both chains of the recombinant bispecific molecules, with a migration corresponding to the theoretical sizes: 77 kDa for the heavy chain, and 25 kDa for the light chain (Figure 2).

The control monospecific antibody, 14F7hT MAb, also showed the presence of bands corresponding to both chains, at the expected sizes: the band of 50 kDa for the heavy chain, and 25 kDa, for the light chain (Figure 2).

### Example 3. BAbs-T recognize NGcGM3 and CD3 molecule.

To determine the dual recognition of the generated antibodies, the reactivity against the ganglioside NGcGM3 and the CD3 molecule was evaluated.

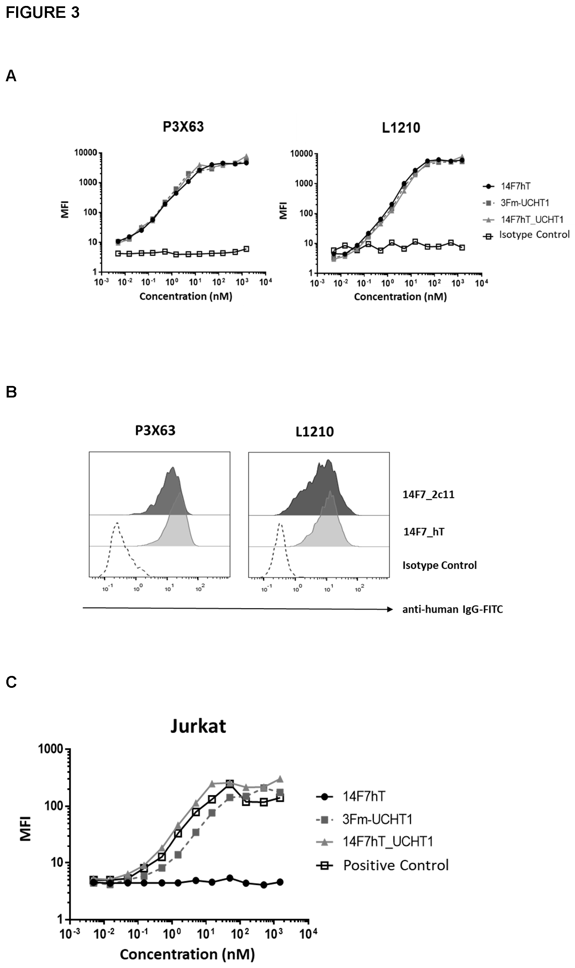

The evaluation of the recognition of the ganglioside by the antibodies 14F7hT-UCHT1 and 3Fm-UCHT1 was determined by their binding to the mouse tumor cell lines L1210 and P3X63, that express high levels of NGcGM3 in their plasma membrane (Carr, A., et al. (2002) Hybridoma hybridomics 21(6):463-468; Roque-Navarro, L., et al. (2008) Molecular Cancer Therapeutics 7(7):2033-2041). This reactivity was determined by flow cytometry and the antibodies were used in equimolar amounts, at different concentrations. As seen in Figure 3A, the BAbs-T 14F7-UCHT1 and 3Fm-UCHT1 demonstrated their ability to recognize both tumor cell lines, in a concentration-dependent manner, as does 14F7hT antibody, used as a positive control. This confirms that the property of the generated recombinant molecules to bind to the antigen in the context of the plasma membrane is not compromised by the presence of the scFv coupled to the carboxyl end of the heavy chain, when compared with a classic IgG of equal specificity. As expected, no recognition of these cells by CC-1, used as an isotype control BAb-T, was observed.

In the case of 14F7hT-2c11 antibody (Figure 3B), its binding to the mouse tumor cell lines P3X63 and L1210 was also evaluated. This recognition was determined by flow cytometry and antibodies were used at 5nM. Its ability to bind to both tumor cell lines was demonstrated, as did the 14F7hT antibody, used as a positive control. As expected, no binding of the BAb-T MOPC-UCHT1, used as an isotype control, was observed.

Taking into consideration that 14F7hT-UCHT1 and 3Fm-UCHT1 are specific for the ganglioside NGcGM3 and the human CD3 molecule, it was also important to determine their ability to interact with the latter, as part of the reactivity study. To this end, its recognition of the human T lymphocyte cell line Jurkat (CD3 positive) was evaluated by flow cytometry. The antibodies were used in equimolar amounts, at different concentrations. Figure 3C shows the dose-dependent binding of the BAbs-T 14F7hT-UCHT1 and 3Fm-UCHT1 to this cell line, in a similar manner to CC-1, with the same format and identical amino acid sequence with respect to the binding site to CD3 (positive control).

### Example 4. Anti-NGcGM3 BAbs-T induce cytotoxicity on ganglioside-positive tumor cell lines.

Considering the previous results, which demonstrate the recognition properties of the 3Fm-UCHT1 and 14F7hT-UCHT1 antibodies, it was decided to evaluate their capacity to mediate cytotoxicity on NGcGM3 positive tumor cell lines. Specifically, P3X63 were selected as target cells (T) for this measurement, and non-activated peripheral blood mononuclear cells (PBMCs) purified from a healthy human donor as effector cells (E), which were co-cultured at a 5:1 (E:T) ratio. The 3Fm-UCHT1 and 14F7hT-UCHT1 antibodies were used in a concentration range of 0.15 to 50 nM and CC-1 was used at 50 nM, as an irrelevant antibody (negative control) with respect to the binding site to the target cell (recognizes the prostate-specific membrane antigen PSMA and the human CD3 molecule, Zekri, L., et al. (2021) EMBO molecular medicine 13(2):e11902). 7-AAD reagent was used to stain dead cells. Cell lysis was measured after 72 hours, by flow cytometry. For each experimental condition, the number of viable target cells (7-AAD- /CD45-) treated with the BAbs-T was counted, and the percentage of specific lysis was calculated using the formula: specific lysis (%) = 100-((number of viable target cells (+BAb-T))/(number of viable target cells (-BAb-T))×100). The evaluations were done with two replicates for each experimental condition.

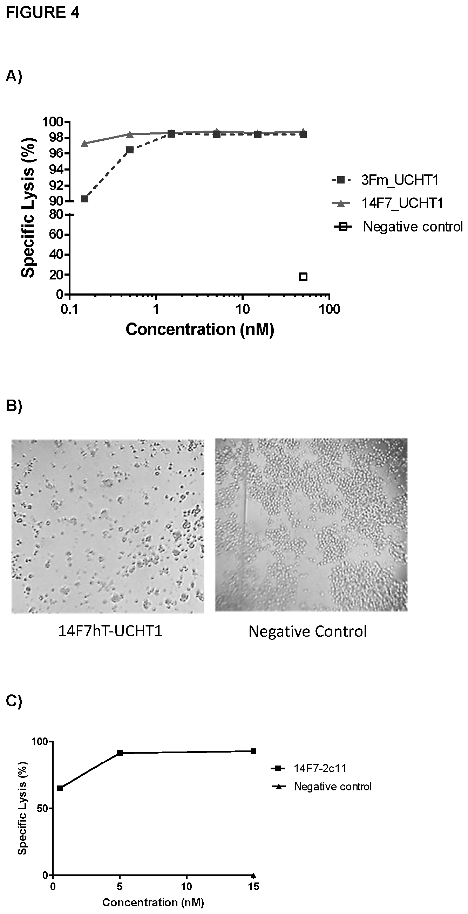

In Figure 4A, a high cytotoxicity (more than 95%) induced by the 14F7hT-UCHT1 and 3Fm-UCHT1 antibodies can be observed, in the presence of effector cells, even at low concentrations such as 0.15 nM. The specificity of this effect is supported by the marginal cytotoxicity (20%) of the irrelevant antibody CC-1 (negative control), evaluated at the maximum concentration tested for the anti-NGcGM3 BAbs-T. As a qualitative measure of this effect, the P3X63 (target) tumor cells, characterized by a larger size, were visualized under an optical microscope, as shown in Figure 4B. Treatment with 14F7hT-UCHT1 (15nM) in the presence of human effector cells, as described above, substantially reduced the number of target cells (left panel), compared to treatment with the irrelevant bispecific antibody (right panel).

For 14F7hT-2c11, its ability to induce cytotoxicity was evaluated on P3X63 mouse myeloma cells (target cells), and as effector cells, T lymphocytes purified from the spleen and lymph nodes of C57BL/6 mice, previously labeled with CFSE. In the cytotoxicity assay, antibodies were used at 15 nM, 5 nM and 0.5 nM. 14F7hT-UCHT1, which recognizes human CD3, was used as an irrelevant antibody (negative control) with respect to the site for the binding to the effector cell. LIVE/DEAD FixableNear-IR Deadcell reagent was used for staining dead cells. Cell lysis was measured after 72 hours by flow cytometry and was calculated using the formula referred to above, based on the number of viable target cells (LIVE/DEAD FixableNear-IR Deadcell-/CFSE-) treated or not with the BAbs-T.

As shown in Figure 4C, the bispecific antibody 14F7hT-2c11 was able to lyse up to approximately 96% of the target cells, in the presence of mouse effector cells. Moreover, the irrelevant antibody respect to binding to mouse T lymphocytes (14F7hT-UCHT1) did not produce this biological effect, which supports the specificity of the cytotoxicity induced by the mouse BAb-T.

### Example 5. BAb-T 14F7hT-2c11 induces cytotoxicity on NGcGM3 positive cell lines mediated by NKT cells.

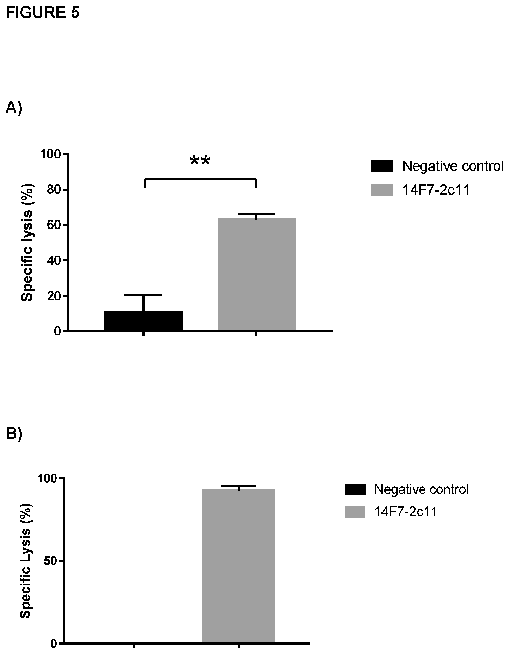

Although the binding of BAbs-T to the CD3 molecule can theoretically also recruit NKT and T^{γδ}, this has only been documented for an antibody with BiTE format, widely recognized for its high potency (Horn, L. A., et al. (2017) Oncotarget 8(35):57964). For this reason, it was decided to evaluate whether an antibody with complete, tetravalent IgG format, such as the one carried by the anti-NGcGM3 BAbs-T of the present invention, can recruit the effector action of NKT lymphocytes on NGcGM3 positive cells. To this end, in a first approach, the ability of 14F7hT-2c11 to mediate the lysis of NGcGM3 positive tumor cells induced by iNKT cells was studied. In this experiment, P3X63 (0.5x10⁵) were incubated as target cells (T), and as effector cells (E), iNKT purified from splenocytes of C57BL/6 mice (Figure 5A), or the FF13 iNKT cell hybridoma (Schümann, J., et al. (2007). European journal of immunology 37 (6): 1431-1441) (Figure 5B), previously labeled with CFSE, which were co-cultured at a 10:1 ratio (E :T). BAbs-T were used at 15 nM. Cell lysis was measured after 72 hours, by flow cytometry. For each experimental condition, the percentage of viable target cells (LIVE/DEAD FixableNear-IR Deadcell-/CFSE-) was calculated, with respect to the condition of untreated tumor cells, using the formula described above. The evaluations were done with two replicates for each experimental condition. 14F7hT-UCHT1, which does not recognize the mouse CD3 molecule, was used as a negative control.

In Figures 5A and B it can be observed that the 14F7hT-2c11 antibody induces cytotoxicity of P3X63 target cells in the presence of iNKT cells, given by a decrease of more than 50% of viable target cells. This result supports another mechanism of action of BAbs-T, barely addressed in the state of the art so far, and not previously described against this target.

### Example 6. 14F7hT-2c11 has an antitumor effect on NGcGM3 positive tumors implanted in immunocompetent mice.

The development of BAbs-T for the clinic has been accompanied by the generation of similar molecules, but specific for tumor antigens and mouse CD3, in order to model the action of this type of biopharmaceuticals in immunocompetent animals (Labrijn, A. F., et al. (2019) Nature reviews Drug discovery 18(8): 585-608; Benonisson, H., et al. (2019) Molecular cancer therapeutics 18(2): 312-322).

For this reason, the antitumor effect of the bispecific antibody 14F7hT-2c11 was measured in a P3X63 therapeutic model, in BALB/c (immunocompetent) mice, to demonstrate the ability to eliminate malignant cells in an in vivo setting, in addition to in vitro, previously tested. To do this, 1 x 10⁶ P3X63 cells were inoculated subcutaneously on the right flank of the animals. Once the tumors were palpable (not measurable: 65 mm³ or 78 mm³), treatments were started, considering this as day 1. Treatments were performed on days 1, 4 and 7 with 12.5 µg of 14F7-2c11 in 200 uL of PBS, intraperitoneally.

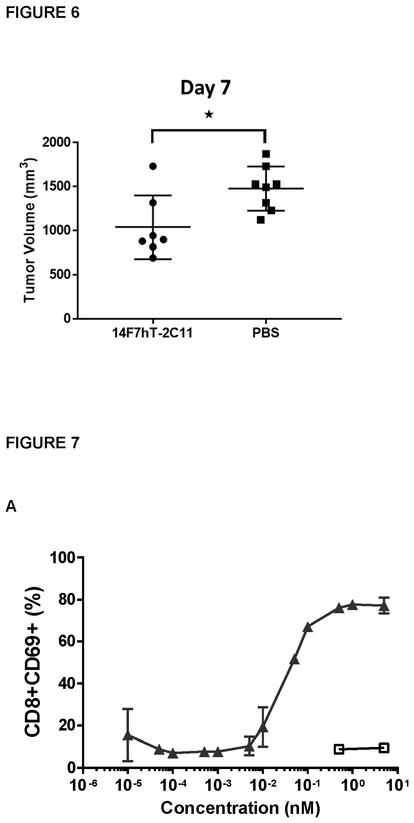

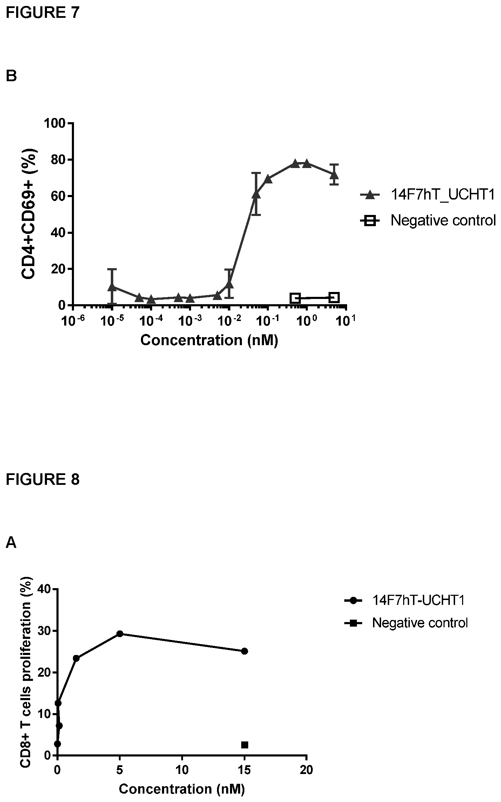

As shown in Figure 6, 7 days after tumor implantation, the group treated with 14F7hT-2c11 showed lower tumor volumes compared to the control group (treated with PBS) (Tukey's test, p<0.05).

This experiment constitutes evidence of the in vivo antitumor effect of 14F7hT-2c11, which validates the therapeutic potential of BAbs-T with the variable region of 14F7hT and the format described above, in a context of immunocompetent individuals.

### Example 7. Anti-NGcGM3 BAbs-T promote T cell activation.

Additionally, as part of the biological activity of the anti-NGcGM3 BAbs-T, it was decided to evaluate the activation status of T lymphocytes. Then, PBMCs from a healthy human donor (source of effector cells) and target cells (P3X63) were co-incubated at a 5:1 ratio, in the presence of the bispecific, at different concentrations and for 72h, and the activation marker CD69 was measured by flow cytometry. As a negative control of the experiment, the same measurement was carried out by co-incubating PBMC with the anti-ganglioside BAb-T, in the absence of tumor cells.

14F7hT-UCHT1 showed a concentration-dependent effect on the activation of CD4+ (Figure 7A) and CD8+ T cells (Figure 7B), based on the expression of the early activation antigen CD69. In contrast, cells treated with BAb-T CC-1 (negative control) showed basal activation percentage values, similar to those of cells without treatment, which also supports the specificity of this effect.

The activation of T lymphocytes by BAbs-T, in the presence of target cells, stimulates their proliferation and expansion, which promotes better antitumor effects. For this reason, we evaluated whether 14F7hT-UCHT1 was capable of promoting a proliferative effect on the CD4+ and CD8+ T cell populations, by co-incubating the PBMCs (2.5x10⁵) labeled with the CTV reagent, with the NGcGM3 positive tumor cell line, P3X63, at a 5:1 (E:T) ratio, and the 14F7hT-UCHT1 antibody, at different concentrations. The bispecific, CC-1 (15 nM) was used as a negative control, while PHA (10 µg/mL) was used as a proliferation positive control. After 72 hours, the dilution of the CTV signal for each T cell subpopulation, indicative of their proliferation, was measured by flow cytometry.

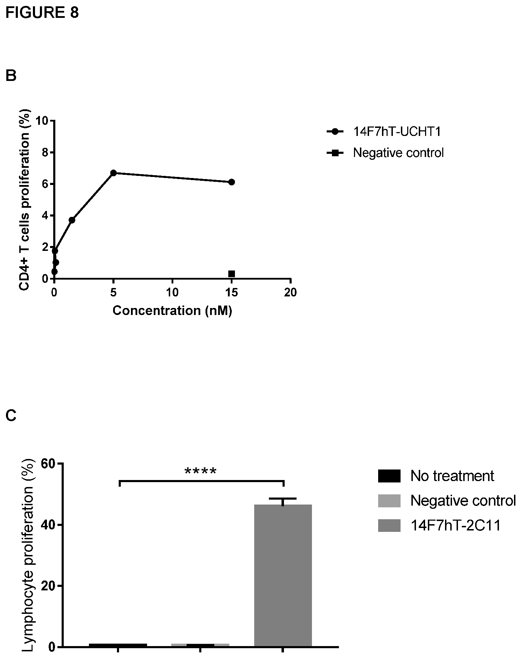

14F7hT-UCHT1 induced the proliferation of human T lymphocytes, preferentially the CD8+ T subpopulation (Figure 8A), which was dependent on the concentration of BAb-T. At the maximum concentration of 15 nM of 14F7hT-UCHT1, 25% of the total CD8+ T lymphocytes initially labeled with the dye were able to proliferate. Similar behavior, but to a lesser extent, was observed in the CD4+ T lymphocyte population (Figure 8B), for which 6% proliferation was verified, at the same concentration. As expected, maximum proliferation was obtained in cells treated with the PHA mitogen, used as a positive control. On the other hand, the irrelevant BAb-T CC-1 (negative control) did not produce this biological effect on T lymphocytes, obtaining percentage values similar to those of untreated cells.

Likewise, we proceeded to evaluate whether 14F7hT-2c11, a surrogate of 14F7hT-UCHT1, was capable of promoting a proliferative effect on mouse T cell populations. To this end, total lymphocytes from C57BL/6 mouse lymph nodes previously labeled with CFSE were co-incubated with the NGcGM3 positive tumor cell line, P3X63, at a ratio of 10:1 (E:T), and the 14F7hT-2c11 antibody at 15nM. The bispecific 14F7hT-UCHT1 (15 nM) was used as a negative control, as it does not recognize mouse T lymphocytes. After 72 hours, the dilution of the CFSE signal for T cells, indicative of their proliferation, was measured using flow cytometry. LIVE/DEAD FixableNear-IR Deadcell was used to measure cell viability. Starting from the LIVE/DEAD FixableNear-IR Deadcell-/CFSE+ cells, the proliferation of T cells was evaluated by quantifying the percentages of T lymphocytes labeled with the CFSE reagent that varied their mean fluorescence intensity (MFI), under the different treatments, in the presence of P3X63 cells. The evaluations were done with three replicates for each experimental condition.

As shown in Figure 8C, with 15 nM of the 14F7hT-2c11 antibody, 45% of the total T lymphocytes initially labeled with the CFSE dye were able to proliferate. In contrast, the irrelevant antibody 14F7hT-UCHT1 did not produce this biological effect on T lymphocytes, obtaining percentage values similar to those of untreated cells (Student's t test, p<0.05).

The set of experiments indicates that the anti-NGcGM3 BAbs-T are capable of inducing the proliferation of T lymphocytes, which contributes to the amplification of the biological effect of these antibodies.

### Example 8. Anti-NGcGM3 BAbs-T only induce cytotoxicity on tumor cells and not on normal NGcGM3 positive cells.

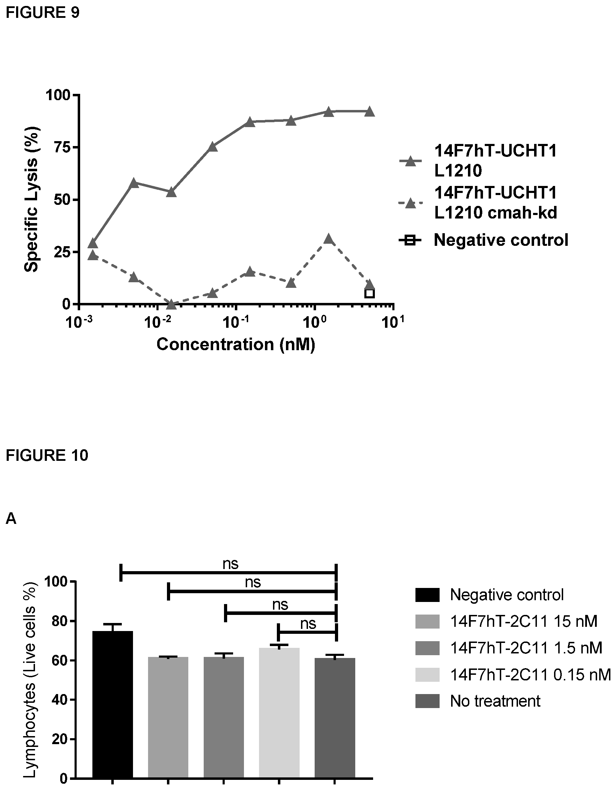

To confirm the specificity of the cytotoxic effect on tumor cells, mediated by anti-NGcGM3 BAbs-T in the presence of effector cells, a study was carried out using the L1210 tumor cell line and its L1210 cmah-kd variant as target cells (T). In this experiment, we proceeded in a similar manner to that described above and used a 2:1 (E:T) ratio, and as a source of effector cells, PBMC from a healthy human donor was used. As can be seen in Figure 9, 14F7hT-UCHT1 was able to induce dose-dependent cytotoxicity on the L1210 cell line, while the viability of the L1210 cmah-kd cells was barely compromised with this treatment. On these cells, the percentages of cytotoxicity were similar to those of L1210 cells treated with the CC-1 antibody (negative control). This result confirms the specificity of the cytotoxic effect induced by this antibody.

Additionally, the high potency of BAb-T 14F7hT-UCHT1 is confirmed here, achieving cytotoxicity greater than 50% with only 10pM of this antibody, and using effectors without prior activation.

Although the presence of the ganglioside is necessary for the cytotoxic action of the T lymphocyte mediated by anti-NGcGM3 BAb-T, the distinction between a normal cell and a tumor cell for this effect is not obvious. As a demonstration of the preferential cytotoxic action of the anti-NGcGM3 BAb-T with this format on tumor cells, compared to normal cells that can express this ganglioside, the lytic effect was evaluated on normal mouse lymphocytes, which are known to have a subpopulation, mostly CD4+, expressing this ganglioside and is recognized by the 14F7 antibody (Roque-Navarro, L., et al. (2008) Molecular Cancer Therapeutics 7(7): 2033-2041).

In order to check whether BAb-T 14F7hT-2c11 can induce cytotoxicity in vitro on these normal cells, total lymphocytes from C57BL/6 mouse lymph nodes (0.5 x10⁶) were incubated with said bispecific, at different concentrations, for 72 h. In this case, T lymphocytes act as effector cells and are also potential target cells, due to the presence of the ganglioside. For each experimental condition, the percentage of viable target cells (LIVE/DEAD FixableNear-IR Deadcell-) was calculated by flow cytometry. As seen in Figure 10A, mouse lymphocytes treated with the 14F7-2c11 antibody at different concentrations do not show differences in the percentage of live cells with respect to those incubated with 14F7-UCHT1 (negative control, which does not recognize the mouse CD3 molecule). This result indicates that the 14F7hT-2c11 antibody only mediates the lysis of tumor cells such as P3X63 (Example 4, Figure 4C), and not of normal cells, such as mouse T lymphocytes.

In a second experiment, total lymphocytes from C57BL/6 mouse lymph nodes (potential target cells) were incubated with 14F7hT-UCHT1 (15 nM), for 72 hours, in the presence of PBMC from a healthy human donor. In this case, for each experimental condition the number of live target cells (mouse lymphocytes) was counted (LIVE/DEAD FixableNear-IR Deadcell-/mouse CD45-), and the percentage of live cells was calculated with respect to the condition of maximum viability, used as reference (mouse lymphocytes incubated with PBMC, without any treatment). The evaluations were done with three replicates for each experimental condition.

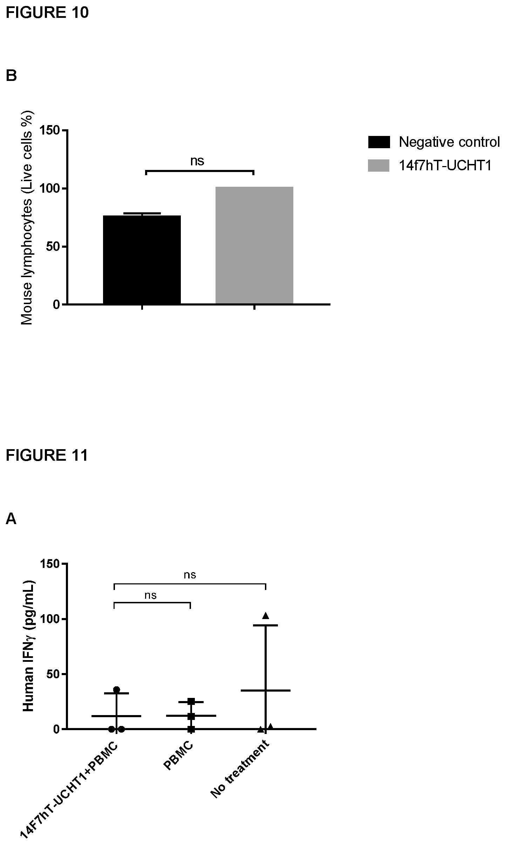

As shown in Figure 10B, there are neither statistically significant differences (Dunn's post-test, p<0.05) between the percentages of live mouse lymphocytes for those that were treated with 14F7hT-UCHT1, compared to those that were incubated with the negative control MOPC-UCHT1 (irrelevant BAb-T, does not recognize the NGcGM3 ganglioside but does recognize the human CD3 molecule, Zekri, L., et al. (2021) EMBO molecular medicine 13(2): e11902). Like 14F7hT-2c11, this result indicates that BAb-T 14F7hT-UCHT1 does not induce lysis on normal cells despite the expression of this ganglioside, unlike tumor cells, on which it does mediate a cytotoxic effect.

### Example 9. Anti-NGcGM3 BAbs-T do not have acute toxicity in vivo.

Once it was demonstrated that the anti-NGcGM3 BAb-T do not lyse normal cells that express said ganglioside, we proceeded to evaluate their possible toxic effect in vivo in a relevant animal model (mice), which naturally express the NGcGM3 ganglioside (Ecsedy, J. A., et al (1999) Journal of neurochemistry 73(1): 254-259). To this end, a study was designed in which BALB/c nu/nu mice were injected on day 1 with 20 µg of 14F7hT-UCHT1, intravenously, and 20x10⁶ human PBMCs, intraperitoneally. This is a dose 10 times higher than that administered for BAbs-T of this format in protocols to evaluate antitumor effect (Zekri, L., et al. (2021) EMBO molecular medicine 13(2):e11902; Mehta, N. K., and cols (2022) Journal for immunotherapy of cancer 10(3):e003882). In addition, a group that only received PBMC and another that did not receive any treatment were included. After 24 hours, serum human IFN-γ levels were examined (Figure 11A), with no statistically significant differences observed between groups. After 48 h, the mice were weighed and sacrificed for subsequent processing of their organs.

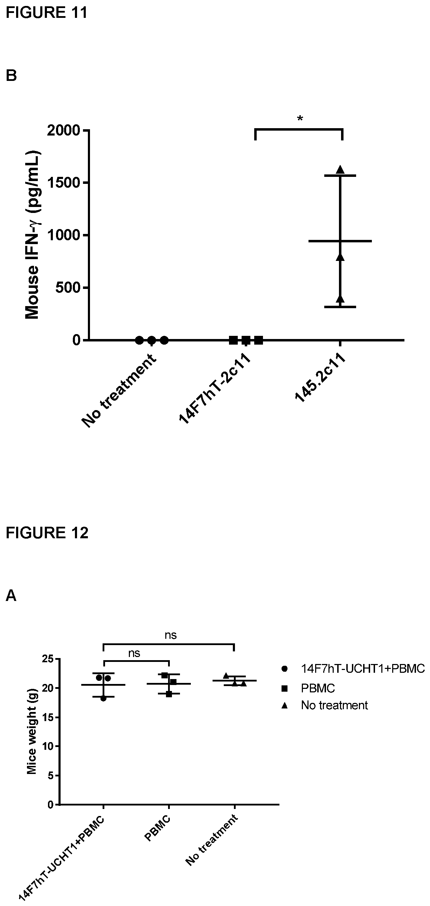

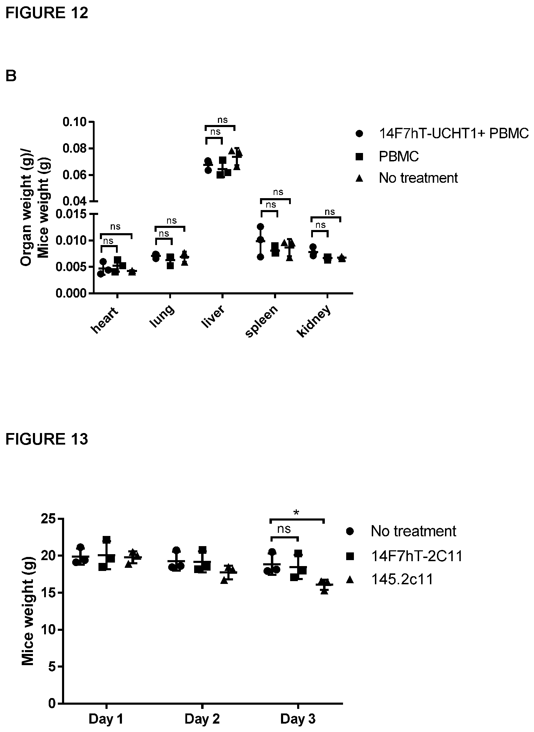

As can be seen in Figure 12 A and B, the treatment with the BAb-T 14F7-UCHT1 and transfer of human PBMC did not induce changes in the body weight of the animals or in the relative weight of the organs, which indicates the little toxicity of the antibody, even in animals that express the ganglioside in their normal cells. Taken together, these results suggest that the application of 14F7hT-UCHT1 together with human PBMC did not induce unwanted T cell activation, in the absence of tumor, and that the antibody is tolerable by the animal, without evidence of acute toxicity.

In a parallel study, the possible toxic effect of the 14F7-2c11 antibody in vivo was evaluated. For this, the 14F7hT-2c11 antibody (50ug) was administered intravenously, while another group received the 145.2c11 antibody (monospecific antibody, anti-mouse CD3, parental of 14F7hT-2c11, which induces T cell activation, Leo, O., et al. (1987) Proceedings of the National Academy of Sciences 84(5):1374-1378), by the same route (positive control).

As seen in Figure 11B, the determination of serum levels of IFN-γ after 24 hours of the application of BAb-T revealed that the 14F7hT-2c11 antibody did not induce secretion of this cytokine in the presence of normal cells that express NGcGM3. This secretion only occurred in mice treated with the anti-mouse CD3 antibody (145.2c11). Additionally, mice were weighed daily during the study. As shown in Figure 13, two days after administration, the group treated with the 145.2c11 antibody decreased in weight (g) with respect to the control group (Tukey's test, p<0.05), unlike that treated with the BAb-T. These findings consolidate the evidence of the activation of anti-NGcGM3 BAb-T only in the presence of the tumor, both in vitro and in vivo. Furthermore, they suggest that "on-target, off tumor" toxicity is minimal, due to non-cytotoxicity on normal cells.

## Claims

1. A bispecific antibody comprising an antibody, antibody fragment or single chain variable fragment (scFv) that recognizes the NGcGM3 ganglioside on tumor cells and an antibody, antibody fragment or scFv that recognizes the human CD3 molecule.

2. The bispecific antibody according to claim 1 wherein the antibody that recognizes NGcGM3 is a human IgG.

3. The bispecific antibody according to claim 1 wherein said antibody has a format that is selected from the group comprising: TandAb, DART, DART-Fc, DuoBody, CrossMab, KiH, BiTE, Triomab, IgG-scFv.

4. The bispecific antibody according to claim 2 wherein the heavy chain is fused directly or through a peptide linker to an antibody, antibody fragment or scFv specific for human CD3.

5. The bispecific antibody according to claim 4 wherein the antibody, antibody fragment or scFv that recognizes the human CD3 molecule has the sequences that are selected from the group comprising SEQ ID NO. 5-7, or variants of these with more than 90% identity with respect to said sequences.

6. The bispecific antibody according to claim 4 wherein the antibody, antibody fragment or scFv that recognizes NGcGM3 has a heavy chain variable region that is selected from the group comprising SEQ ID NO. 10 and SEQ ID NO. 15, or variants of these with more than 90% identity with respect to these sequences.

7. The bispecific antibody according to claim 4 wherein the antibody, antibody fragment or scFv that recognizes NGcGM3 has a light chain variable region that is selected from the group comprising SEQ ID NO. 11-14 or variants of these with more than 90% identity with respect to these sequences.

8. The bispecific antibody according to claim 2 wherein the human IgG sequence is selected from the group comprising SEQ ID NO. 1-4 or variants of these with more than 90% identity with respect to these sequences.

9. A pharmaceutical composition comprising as active ingredient the bispecific antibody of any of claims 1-8 and a pharmaceutically acceptable carrier.

10. Use of the bispecific antibody of any of claims 1-8 in the treatment of lymphoproliferative disorders and solid tumors that express NGcGM3.

11. Use of the nucleic acid encoding BAbs-T according to any of claims 1-8 in the gene therapy of tumors that express NGcGM3.

12. The use according to claim 11 wherein the gene therapy is based on the injection of mRNA or transducer particles encoding the BAbs-T.

13. A method of treating a subject in need thereof that comprises the administration by subcutaneous, intravenous, intradermal, intramuscular, intratumoral or intraperitoneal route of the bispecific antibody of any of claims 1-8 in a dose range between 20ug-10mg.
